# EUROPEAN PATENT APPLICATION

(11) **EP 1 932 473 A1**
(43) Date of publication of application: **18.06.2008**
(21) Application number: 06810073.4
(22) Date of filing: 13.09.2006
(51) Int. Cl.: A61B 5/08, A61B 5/11

(54) **COUGH DETECTING DEVICE AND COUGH DETECTING METHOD**

(30) Priority: 05.10.2005 JP 2005292085
(71) Applicant: Konica Minolta Medical & Graphic, Inc., Hino-shi, Tokyo 191-8511 (JP)
(72) Inventor: WADA, Yasunori, Tokyo 163-0512 (JP)
(74) Representative: Alton, Andrew
(86) International application number: PCT/JP2006/318108
(87) International publication number: WO 2007/040022

(57) **Abstract**

A cough detecting apparatus capable of high-precision couching detection is provided. This cough detecting apparatus includes a voice measuring section for detecting the voice of a subject, a body motion measuring section for detecting the body motion of the subject, and a cough detecting section for detecting coughing, based on the voice detected by the voice measuring section and the body motion detected by the body motion measuring section.

## Description

### TECHNICAL FIELD

The present invention relates to a cough detecting apparatus and cough detecting method.

### BACKGROUND

Coughing is often observed in the respiratory system disease (e.g., particularly, asthma, chronic obstructive pulmonary disease and bronchitis). At present, diagnosis of coughing depends on a medical examination by interview with a doctor. However, a patient is not always coughing at the time of medical examination. There is no other way for the doctor but to inquire about the subjective symptom of the patient. Further, although the patient is conscious of the symptom in the daytime when he is awake, details of coughing experienced during the sleep cannot be identified. The information that can be obtained will be limited to such a statement that the coughing was severe or that the patient could not sleep due to severe coughing. Thus, objective evaluation cannot be conducted, with the result that effective medical treatment cannot be provided, according to the conventional method.

To conduct objective evaluation of coughing, the apparatuses are disclosed wherein voice signal information characteristic of coughing is stored in advance, and based on the voice signal information characteristic of coughing, a voice signal resulting from coughing is identified and extracted from the voice signal inputted from a microphone and others, whereby coughing is detected and monitored (e.g., Patent Documents 1 through 3).
[Patent Document 1] Unexamined Japanese Patent Application Publication (Tokkaihei) No. 7-376
[Patent Document 2] Unexamined Japanese Patent Application Publication (Tokkaihei) No. 8-38481
[Patent Document 3] Unexamined Japanese Patent Application Publication (Tokkai) No. 2003-38460

### DISCLOSURE OF THE INVENTION

### PROBLEMS TO BE SOLVED BY THE PRESENT INVENTION

However, the apparatus disclosed in the aforementioned Patent Document cannot easily distinguish between coughing and a similar sound when a sound similar to coughing occurs. Such a sound may be detected as coughing. Further, the noise caused by being in contact with a sensor for sampling the voice such as a microphone or piezoelectric device cannot be always distinguished from coughing. When a patient is not coughing, coughing by another person may be captured by the microphone and may be identified as the patient's coughing.

The object of the present invention is to solve the aforementioned problem and to provide a cough detecting apparatus and a cough detecting method capable of detecting coughing with high precision.

### MEANS TO SOLVE THE PROBLEMS

The cough detecting apparatus of the present invention includes:
a voice measuring section for detecting subject's voice;
a body motion measuring section for detecting the body motion of the subject; and
a cough detecting section for detecting coughing based on the voice information detected by the aforementioned voice measuring section, and the body motion information detected by the aforementioned body motion measuring section.

The cough detecting method of the present invention includes:
a voice measuring step for measuring subject's voice using a voice measuring section;
a body motion measuring step for measuring the subject's body motion using a body motion measuring section;
a cough detection step wherein the voice information measured by the aforementioned voice measuring step and the body motion information measured by the aforementioned body motion measuring step are inputted into the cough detecting section to detect coughing.

### EFFECTS OF THE INVENTION

When detecting the sound of coughing, according to the present invention, the body motion (movement of the subject's body) is also detected being associated therewith. A sound similar to coughing having been detected is identified as a non-coughing sound if the body motion inherent to coughing has not been detected. This arrangement ensures high-precision coughing detection.

### BRIEF DESCRIPTION OF THE DRAWINGS

Fig. 1 is a schematic structural diagram showing a cough detecting apparatus of the present embodiment.
Fig. 2 is a flow chart representing a process of cough measurement of the present embodiment.
Fig. 3 is a conceptual diagram representing the voice data and body motion data relating to a second embodiment.
Fig. 3(a) is a diagram representing the voice data Sv(a) and body motion data Sm(a) measured by the cough detecting apparatus of the present embodiment when a subject has coughed.
Fig. 3(b) is a diagram showing the voice data Sv(b) and body motion data Sm(b) when another person close to the subject has coughed.
Fig. 4 is a flow chart showing a process of coughing detection of the present embodiment.
Fig. 5 is a diagram showing an example of the body motion data of the abdominal region (below the diaphragm) measured by a body motion measuring section 20.
Fig. 6 is a conceptual diagram showing the concept of a method of extracting the body motion waveform characteristic of coughing.
Fig. 6(a) is a diagram showing the data on body motion caused by coughing.
Fig. 6(b) is a diagram showing the time durations T1, T2, and T3.
Fig. 6(c) is a diagram showing the inclination angle θ1 and θ2.
Fig. 6(d) is a diagram showing the kurtosis K.

### EXPLANATION OF THE SYMBOLS

10 Voice measuring section
11 Microphone
20 Body motion measuring section
21 Accelerometer
30 Cough detecting section

### DESCRIPTION OF THE PREFERRED EMBODIMENT

### (Apparatus Configuration)

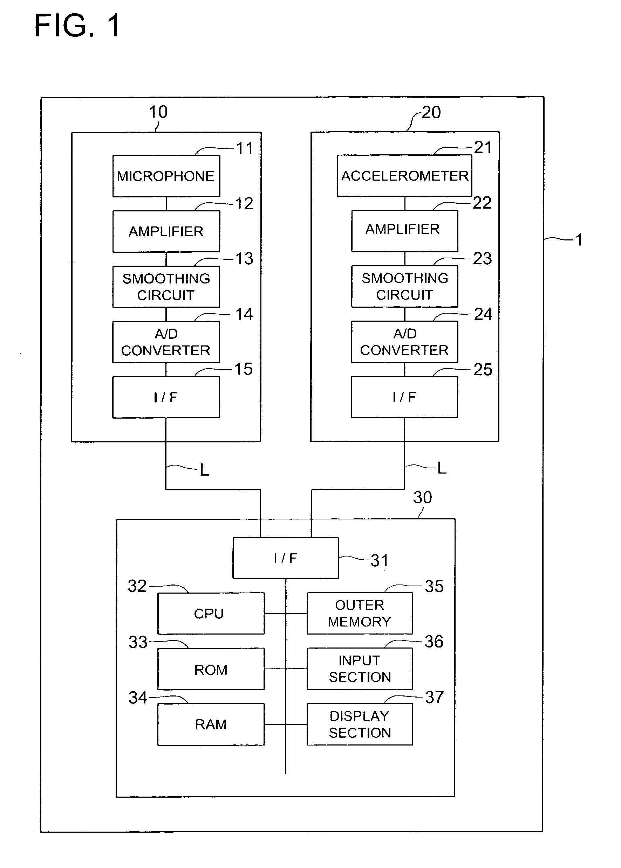

Fig. 1 is a structural diagram showing a cough detecting apparatus of the present embodiment. The cough detecting apparatus 1 is composed of a voice measuring section 10, a body motion measuring section 20 and a cough detecting section 30. The voice measuring section 10 and body motion measuring section 20 each are connected to the cough detecting section 30 via a communication medium L. This communication medium L can be either wired or wireless means. If wireless means is used for connection, it is possible to reduce the possibility of giving unwanted vibration or noise to a sensor used for the voice measuring section 10 and body motion measuring section 20 through a wire, which happens in the case of wired means. This arrangement minimizes the restriction of the subject behavior.

The voice measuring section 10 includes a microphone 11 for converting the inputted voice into a voice signal; an amplifier 12 for amplifying the voice signal converted by the microphone 11; a smoothing circuit 13 for half-wave rectification and smoothing of the voice signal amplified by the amplifier 12; an A/D converter 14 for converting the voice signal processed by the smoothing circuit 13, into voice data; and an I/F 15 for transmitting the voice data having been converted by the A/D converter 14 to the cough detecting section 30.

When sound is measured using the microphone 11 as in the present embodiment, noise will be generated if the human body and clothing are brought into contact with the microphone. This requires the microphone to be fixed not to come in contact with them. However, if the microphone is brought too far away from the human body, there will be an increase in the influence of external noise. Thus, the microphone is preferably placed as close as possible to the human body.

In addition to the microphone, a piezo microphone or accelerometer can be placed in contact with the pharyngeal part or others, and the sound can be detected as vibration. In this case, since the equipment is kept in contact with the human body, it is not much affected by external noise.

The body motion measuring section 20 includes an accelerometer 21 for converting the inputted body motion into a body motion signal; an amplifier 22 for amplifying the body motion signal converted by the accelerometer 21; a filter circuit 23 for removing a low-frequency component below a predetermined frequency and a high-frequency component above a predetermined frequency from the body motion signal amplified by the amplifier 22; an A/D converter 24 for converting the body motion signal processed by the filter circuit 23 into the body motion data; and an I/F 25 for transmitting to the cough detecting section 30 the body motion data having been converted by the A/D converter 24.

The accelerometer 21 for measuring the body motion is preferably located at the abdominal region, chest region or cervical region, more preferably at the abdominal region or chest region. This is because the region around the diaphragm or chest region exhibits a characteristic movement at the time of coughing.

The accelerometer 21 is preferably attached onto the skin directly or indirectly by a double-faced tape coated with adhesive or the like, which is less sensitive to the skin. Further, noise preventive covering material or cushioning material is preferably arranged around the accelerometer 21.

The cough detecting section 30 includes an I/F 31 for receiving the voice data and body motion data from the voice measuring section 10 and body motion measuring section 20; a CPU 32 for processing the voice data and body motion data received by the I/F 31 according to the program; a ROM 33 for storing the program and data required by processing by the CPU 32; a RAM 34 for temporarily storing the program and data required for processing by the CPU 32; an outer memory 35 for storing the result of processing by the CPU 32, into the hard disk, DVD-R or CD-R; an input section 36 for inputting data into the cough detecting section 30; and a display section 37 for displaying the result of processing by the CPU 32.

The cough detecting section 30 may be formed of a special-purpose information processing apparatus or a general-purpose personal computer. In the case of a personal computer, use of an easy-to-carry portable information terminal (PDA) is preferred.

In the present embodiment, each of the voice measuring section 10 and body motion measuring section 20 is provided with an A/D converter 14 and A/D converter 24 respectively. Further, the cough detecting section 30 may be provided with an A/D converter.

### (Cough measurement)

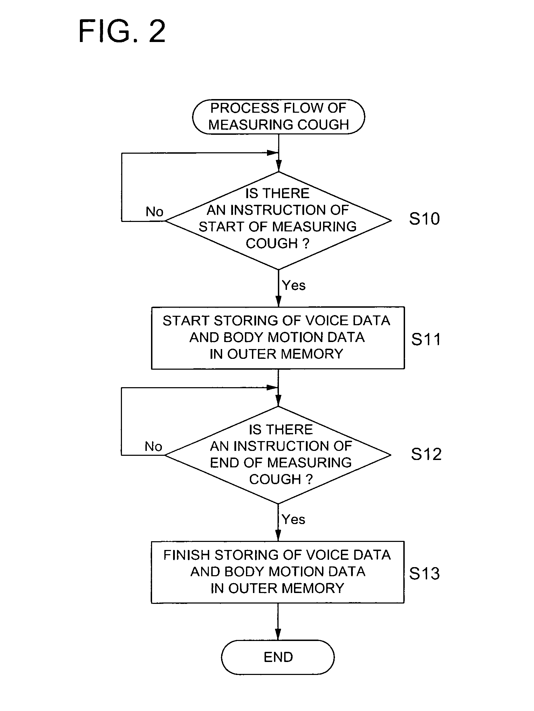

Fig. 2 is a flow chart representing a process of cough measurement of the present embodiment. This cough measurement flow is executed by the CPU 32 based on the cough measurement program in the ROM 33. In this case, it is assumed that the ID and other information for identifying the subject has been inputted by the input section 36 in advance.

In the first place, the CPU 32 takes a decision step to determine whether or not a cough measurement start instruction has been issued by the input section 36 (Step S10). If decision has been made that start of cough measurement has been specified (Step S10: Yes), the CPU 32 initiates the step of storing the voice data and body motion data from the voice measuring section 10 and body motion measuring section 20 into the outer memory 35 via the RAM 34 (Step S11). In this case, voice data and body motion data are stored being associated with the subject ID and the time. If decision has been made that a cough measurement start instruction has not been issued (Step S10: No), the control goes back to the Step S10 to wait until a cough measurement start instruction is given.

Then the CPU 32 takes a decision step to determine whether or not a cough measurement termination instruction has been issued (Step S12). If it has been determined that a cough measurement termination instruction has been issued (Step S12: Yes), the CPU 32 terminates the step of storing the voice data and body motion data from the voice measuring section 10 and body motion measuring section 20 into the outer memory 35 via the RAM 34 (Step S13). If it has been determined that a cough measurement termination instruction has not been issued (Step S12: No), the control goes back to the Step S42 to wait until the cough measurement termination instruction is given.

### (Coughing voice signal and body motion signal)

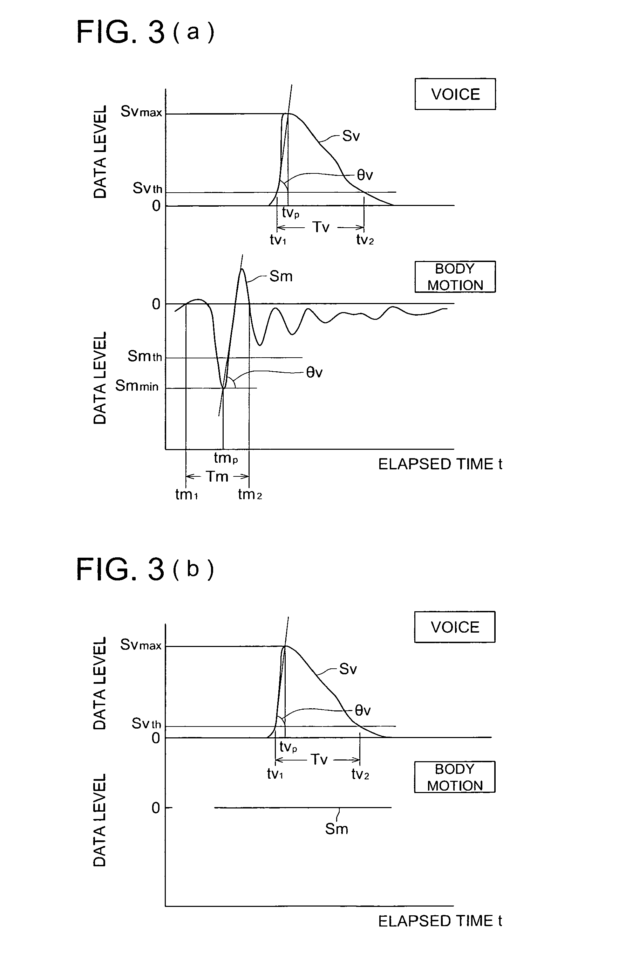

Fig. 3 is a conceptual diagram representing the voice data Sv and body motion data Sm of the present invention. The voice data Sv and body motion data Sm in Fig. 3 represent the data outputted from the voice measuring section 10 and body motion measuring section 20.

Fig. 3(a) shows the voice data Sv(a) and body motion data Sm(a) measured by a cough detecting apparatus of the present embodiment when the subject has coughed, wherein elapsed time is plotted on the horizontal axis, and data level is plotted on the vertical axis in a schematic representation. Coughing is produced by a sudden exhalation of the air stored in the abdominal region subsequent to closure of the throat. A strong noise is produced in a short period of time, and the abdominal region is dented in a short period of time too. Thus, as illustrated, when coughing occurs, both the rising gradient θv(a) of the voice data Sv(a) and θm(a) of the motion data Sm(a) are steep, and both the time width Tv(a) and Tm(a) are short.

Fig. 3(b) is a diagram showing the voice data Sv(b) and body motion data Sm(b) when another person close to the subject has coughed. As illustrated, the rising gradient θv(b) of the voice data Sv(b) is detected similarly to the case of Fig. 3(a), but the rising gradient of the body motion data Sm(b) is not detected.

To be more specific, to determine if the subject has coughed or not, it is effective to check that each of the rising gradient θv of voice data Sv and θm of body motion data Sm is equal to or greater than a predetermined value, and each of the time width Tv and Tm does not exceed a predetermined value.

### (Cough detection)

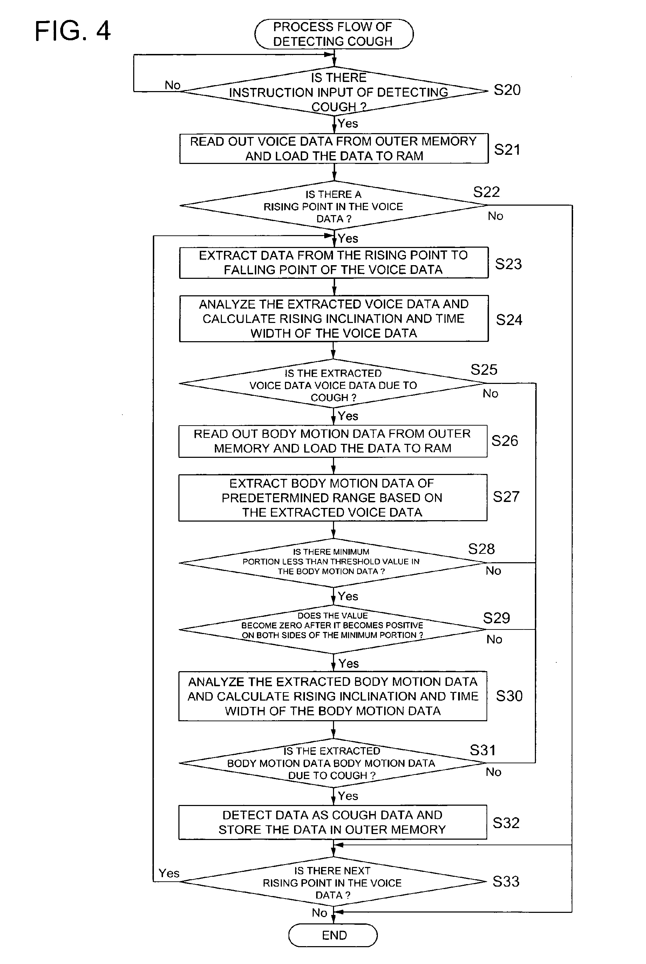

Fig. 4 is a flow chart showing a process of coughing detection of the present embodiment. This cough detection flow is executed by the CPU 32 based on the cough detection program in the ROM 33. In this case, it is assumed that the ID and other information for identifying the subject has been inputted by the input section 36 in advance.

In the first place, the CPU 32 determines whether or not a cough detection instruction has been inputted from the input section 36 (Step S20). If decision has been made that the cough detection instruction has been inputted (Step S20: Yes), the CPU 32 reads out the voice data corresponding to the subject ID from the outer memory 35, and load it into the RAM 34 (Step S21). If decision has been made that the cough detection instruction has not been inputted (Step S20: No), the control goes back to the Step S20 to wait until the cough detection instruction is inputted.

Then the CPU 32 analyzes a temporal change of the voice data stored in the RAM 34. A decision step is taken to determine whether or not the rising point (tv1 of Fig. 3(a)) is included in the voice data wherein the voice level rises from the level below the threshold value level (Svth of Fig. 3(a)) to the level above the threshold value level (Step S22). If it has been determined that the rising point is included in the voice data (Step S22: Yes), the CPU 32 extracts the voice data from the rising point tv1 of the voice data to the falling point of the voice (tv2 of Fig. 3(a)) wherein the voice level is reduced from the level above the threshold value level to the level below the threshold value level (Step S23). If it has been determined that the voice data includes no rising point (Step S22: No), the flow terminates.

Then the CPU 32 analyzes the voice data extracted in the Step S23, and calculates the rising gradient θv of the voice level, time width Tv, and the maximum voice level Svmax (Step S24).

Then the CPU 32 determines whether or not the rising gradient 8v of the voice level obtained by calculation and the voice data extracted from the time width Tv are the voice data resulting from coughing. If the rising gradient 8v of the voice level is equal to or greater than a predetermined value θv0 and time width Tv is below a predetermined value Tv0, they are determined to be the voice data resulting from coughing. Otherwise, they are determined not be the data resulting from coughing (Step S25).

If it has been determined that the extracted voice data is the data resulting from coughing (Step S25: Yes), the CPU 32 reads the body motion data corresponding to the subject ID from the outer memory 35, and loads it into the RAM 34 (Step S26). If it has been determined that the voice data is not voice data resulting from coughing (Step S25: No), the control jumps to the Step S33.

From the body motion data loaded into the RAM 34 in the Step S26, the CPU 32 extracts the body motion data within a predetermined range determined based on the aforementioned extracted voice data (Step S27). Since during coughing the voice is generated after the movement of the abdominal region and throat region, the body motion is detected earlier than the voice. Thus, the body motion data should be extracted a predetermined time earlier than the rising point tv1 in the voice data. For example, the body motion data are sufficient only when they are extracted from the time which is about 200 msec earlier than the rising point time tv1 of the voice data up to the falling point time tv2 of the voice data.

Then the CPU 32 analyzes the extracted body motion data and determines whether or not the body motion data includes the minimum value Smmin which does not exceed the threshold value Smth (Step S28). If it has been determined that the body motion data includes the minimum value Smmin which does not exceed the threshold value Smth (Step S28: Yes), a step is taken to determine whether or not the data level turns once positive, and then goes to 0 (wherein the time when 0 is reached is represented by tm1 and tm2) when the time is traced, toward both before and after, from the time tmp at which this minimum value Smmin is obtained. To be more specific, a decision step is taken to determine whether or not the waveform is characteristic of coughing (Step S29). The details will be described later.

A step is taken to trace the time toward both before and after from the time tmp at which the minimum value Smmin is obtained. If it has been determined that the data level turns once positive, and then goes to 0 (Step S29: Yes), the CPU 32 analyzes the extracted body motion data, and calculates the rising gradient θm from the minimum value Smmin of the body motion level, the time width Tm from tm1 to tm2, and others (Step S30).

A step is taken to trace the time toward both before and after from the time tmp at which the minimum value Smmin is obtained. If it has been determined that the data level has not turned positive to later become 0 (Step S29: No), the CPU 32 jumps to Step S33.

Based on the rising gradient θm from the minimum value Smmin of the body motion level obtained by calculation in Step S30, and the time width Tm from tm1 to tm2, the CPU 32 determines whether or not the body motion data is the body motion data resulting from coughing. If the rising gradient θm from the minimum value Smmin of the body motion level is equal to or greater than a predetermined value θm0, and the time width Tm from tm1 to tm2 does not exceed a predetermined value Tm0, then the extracted body motion data is determined to be the body motion data resulting from coughing. Otherwise, this data is determined not to be the body motion data resulting from coughing (Step S31).

If the extracted body motion data has been determined to be the body motion data resulting from coughing (Step S31: Yes), the CPU 32 detects the extracted voice data under the conviction that the data results from coughing, and stores the data into the outer memory 35 (Step S32). In this case, the time of occurrence t, the maximum voice level Svmax, the minimum body motion level Smmin and others are stored being associated with the subject ID. Further, the pattern of at least one of voice data and body motion data corresponding to the type of coughing is stored in the ROM 33 or outer memory 35. The type of coughing is identified by comparison with the measured pattern corresponding to the aforementioned pattern, and can be stored being associated with the subject ID. If the extracted body motion data has been determined not to be the body motion data (Step S31: No), the control jumps to Step S33.

In Step S33, the CPU 32 analyzes the temporal change of the voice data after the aforementioned extracted voice data loaded in the RAM 34. The CPU 32 takes a decision step to determine whether or not the rising point tv1 is included in the voice data wherein the voice level has increased from the level below the threshold value level Svth to the level above the threshold value level Svth. If the voice data has been determined to include the rising point tv1 (Step S33: Yes), the control goes back to Step S23, and cough detection is repeated. This means that cough detection has been performed in all the area including the rising point tv1. If the voice data is determined not to include the rising point tv1 (Step S33: No), the flow terminates.

As described above, when detecting coughing, according to the present embodiment, it is detected being associated with the body motion. Even if the voice similar to coughing has been found out, this arrangement determines that it not coughing, if the body motion inherent to coughing has not been detected. Thus, the present embodiment ensures high-precision detection of coughing.

### (Method of extracting body motion waveform characteristic of coughing)

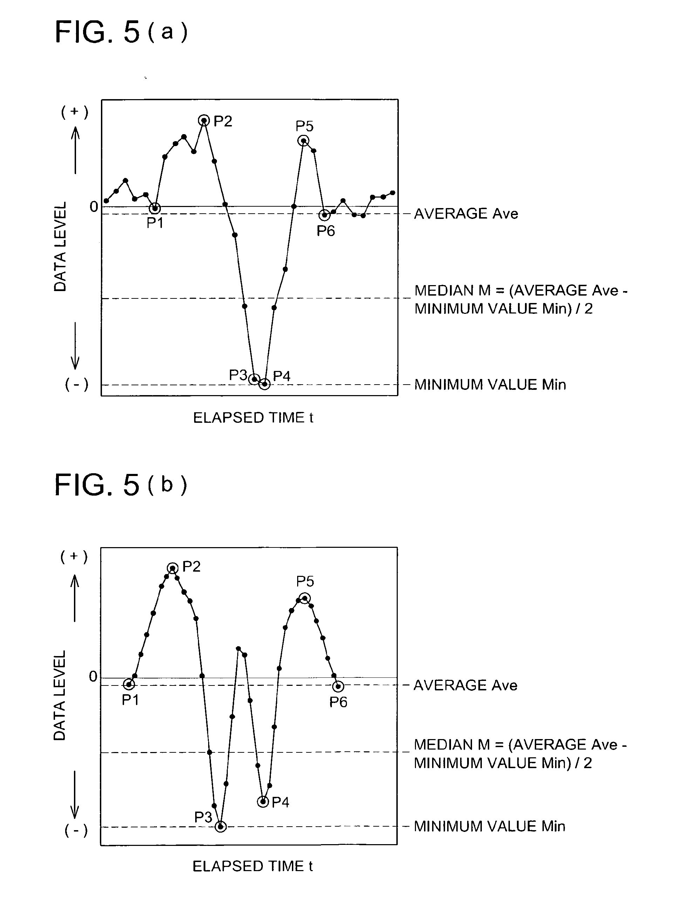

Fig. 5 (a) shows an example of body motion data resulting from coughing. The data level starts to rise in the positive direction at P1 to reach the maximum at P2. In a previous stage to coughing, the vocal cord is closed and the intrapleural pressure rises. The diaphragm lowers, and the abdominal region protrudes. This is followed by an abrupt fall in the negative direction to reach P3 and P4. The vocal cord is opened suddenly. Then exhalation and coughing occur at the same time so that the diaphragm rises and the abdominal region retracts. After that, there is a sudden rise from P4 in the positive direction to reach P5. The diaphragm is lowered by the reaction of exhalation, and the abdominal region protrudes. After that, data level falls toward P6. The diaphragm is raised by the reaction in the reverse direction and the abdominal region retracts. As described above, after exhalation of coughing, the movement of the diaphragm fades out by repeating up and down reaction.

The diagram shows the average value Ave, the minimum value Min, and the median M which is the average value between the average value Ave and the minimum value Min ((the average value Ave + the minimum value Min) / 2), which are data levels used in the process of coughing detection to be described later.

Some type of coughing exhibits a waveform wherein there is a rise in the positive direction between P3 and P4, as shown in Fig. 5 (b).

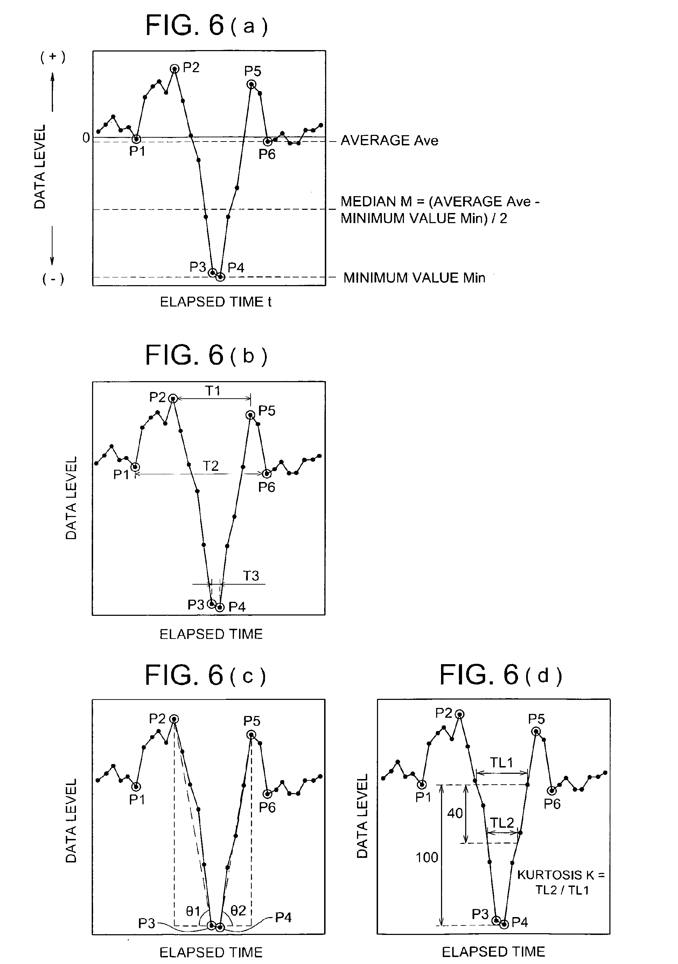

Fig. 6 is a conceptual diagram showing the concept of a method of extracting the body motion waveform characteristic of coughing. Fig. 6 (a) is a diagram showing the data on body motion caused by coughing, as shown in Fig. 5 (a). The following description assumes that the body motion data of Fig. 6 (a) has been obtained in a certain sampling frame. The preferable width of the sampling frame is equivalent to the time when the data from P1 to P6 are included, as shown in Fig. 6 (a), namely, about 200 msec through 400 msec is preferred. The preferred sampling interval within the sampling frame W is about 3 through 20 msec when consideration is given to the detection precision and load of calculation.

In the first place, the average value Ave of the data level of the sampling data in the sampling frame is calculated. In the sampling frame, the sampling point of the minimum value Min (P4 in Fig. 6 (a)) of the data level is identified. The next step is to calculate the average value between the average value Ave and the minimum value Min of the data level (hereinafter referred to as "median M"). This is followed by the step of detecting if there is a sampling point denoting the minimal value smaller than the median M, in addition to the sampling point denoting the minimum value Min. In Fig. 6 (a), there is no sampling point denoting the minimal value smaller than the median M, other than the sampling point P4 denoting the minimum value Min. In the waveform shown in Fig. 5 (b), there is a sampling point P4 (a lower convex on the right in the diagram) denoting the minimal value smaller than the median M, in addition to the sampling point P3 denoting the minimum value Min.

If there is a sampling point denoting the minimal value as in Fig. 5 (b), it is assumed that among the sampling point denoting the minimum value Min and the sampling point showing the minimal value, which is located on the left, is P3, and which is located on the right, is P4. If there is no sampling point denoting the minimal value as shown in Fig. 5 (a) and Fig. 6 (a), a step is taken to identify the sampling point wherein there is a sudden change in value on the right side and left side of the sampling point denoting the minimum value Min in the vicinity of the sampling point, including the sampling point. In Fig. 6 (a), P3 is extracted as the sampling point wherein there is a sudden change of value on the left side, and P4 is extracted as the sampling point wherein there is a sudden change of value on the right side. In some cases, the sampling point wherein there is a sudden change of value on the left side is the same as the sampling point wherein there is a sudden change of value on the right side. In this case, P3 agrees with P4.

The aforementioned procedure is followed by the step of identifying the sampling point of the maximum value toward the left from the sampling point P3, and sampling point of the maximum value toward the right from the sampling point P4. In Fig. 6 (a), P2 is extracted as the sampling point of the maximum value toward the left from the sampling point P3, whereas P5 is extracted as the sampling point of the maximum value toward the right from the sampling point P4.

The next step is to identify the sampling point wherein the value has a sudden decrease to reach close to 0 toward the left side from the sampling point P2, and the sampling point wherein the value has a sudden decrease to reach close to 0 toward the right side from the sampling point P5. In Fig. 6 (a), P1 is extracted as the sampling point wherein the value has a sudden decrease to reach close to 0 toward the left side from the sampling point P2, and P6 is extracted as the sampling point wherein the value has a sudden decrease to reach close to 0 toward the right side from the sampling point P5.

### (Another method of determining if the body motion waveform characteristic of coughing results from coughing or not)

Fig. 6 (b) is a diagram showing the time duration T1 from P2 to P5, time duration T2 from P1 to P6, and time duration T3 from P3 to P4. At least one of the time duration T1 and T2 serves as a parameter to determine if the body motion results from coughing. As time durations T1 and T2 are shorter, there is a greater possibility of being determined as coughing. The time duration T3 varies according to the type of coughing. It can be used to determine the type of coughing.

Fig. 6 (c) indicates the inclination angle θ1 of the line segment joining the points P3 and P2, and the inclination angle θ2 of the line segment joining the P4 and P5. The greater of the inclination angles θ1 and θ2 is designated as the maximum inclination angle θmax. This maximum inclination angle θmax serves as a parameter to determine if the phenomenon is from coughing or not. The greater the maximum inclination angle θmax, the greater the probability of being determined as coughing.

Instead of the maximum inclination angle θmax of Fig. 6 (c), kurtosis K shown in Fig. 6 (d) can be used as a parameter. For example, a valley-shaped waveform leading from point P2 to point P5 through points P3 and P4 is assumed. The ratio (TL2/TL1) is defined as kurtosis K wherein the time TL1 is the width of the valley at the data level of the sampling point P1 and the time TL2 is the width of the valley at the data level 40 lower than the data level of the sampling point P1 when the difference between the data level of the sampling point P1 and the data level of P4 of the minimum value is assumed as 100. The smaller the kurtosis K, the greater the possibility of being determined as coughing.

At least one of the time durations T1 and T2 of Fig. 6 (b) and the maximum inclination angle θmax of Fig. 6 (c) or the kurtosis K of Fig. 6 (d) are used as parameters to practice the multivariate analysis method. Then a decision is made to see if the body motion data of the targeted sampling frame results from coughing or not. The intensity of coughing can be determined, for example, by the data of P3 or P4 as the minimum value of the data level in the sampling frame in which a coughing is determined. Further, the time duration T1, T2 and T3 can be used to identify the type of coughing.

In the present embodiment, if the voice data was determined as resulting from coughing after analysis of voice data, the body motion data was analyzed to detect coughing. Conversely, if the body motion data is determined to have resulted from coughing by the analysis of the body motion data, coughing can be detected by the analysis of voice data. It is also possible to make such arrangements that voice data and body motion data are analyzed independently of each other. If both are determined to have resulted from coughing, coughing is detected.

Further, coughing can be detected, for example, by the computation such as multiplication of voice data by body motion data. If the data results from coughing, as described above, the portion of the voice data output (portion where voice is detected) and the portion of the body motion data output (portion where body motion is detected) have a predetermined time lag. Accordingly, if the data results from coughing, they are shifted by the predetermined time lag. This will bring about agreement between the portion of voice detection and that of body motion detection. Conversely, if the data does not result from coughing, the portion of voice data output (portion of voice detection) is uncorrelated with that of body motion data output (portion of body motion detection), and there is a high probability that they do not agree with each other.

The aforementioned description suggests that the data subsequent to the computation of multiplication is outputted over a relatively long time when the data results from coughing, as compared to the case where the data does not result from coughing. Thus, if the computation of multiplication is performed, whether or not the data results from coughing can be determined by whether or not the time of output of the data subsequent to computation is longer than a predetermined time.

## Claims

1. A cough detecting apparatus comprising:
a voice measuring section for detecting a voice of a subject;
a body motion measuring section for detecting a body motion of the subject; and
a cough detecting section for detecting coughing, based on voice information detected by the voice measuring section and body motion information detected by the body motion measuring section.

2. The cough detecting apparatus of claim 1, wherein the cough detecting section comprises;
a first determining section for determining if coughing has occurred or not, based on the voice information detected by the voice measuring section; and
a second determining section for determining if coughing has occurred or not, based on the body motion information detected by the body motion measuring section;
wherein, when both the first determining section and the second determining section determine that coughing has occurred, it is determined that the cough detecting section has detected coughing.

3. The cough detecting apparatus of claim 1
wherein the cough detecting section detects coughing by computation based on the voice information detected by the voice measuring section and the body motion information detected by the body motion measuring section.

4. The cough detecting apparatus of any one of claims 1 - 3, wherein the body motion measuring section includes an accelerometer.

5. The cough detecting apparatus of any one of claims 1 - 4, wherein the body motion measuring section includes a body motion sensor to be mounted on an abdominal region or a chest region.

6. A cough detecting method comprising steps of:
measuring a voice of a subject by a voice measuring section;
measuring a body motion of the subject by a body motion measuring section; and
detecting coughing by inputting voice information measured in the voice measuring step and body motion information measured in the body motion measuring step into a cough detecting section.

7. The cough detecting method of claim 6, wherein the coughing detecting step comprises steps of:
first determining if coughing has occurred or not, based on the voice information;
second determining if coughing has occurred or not, based on the body motion information;
wherein, when in both the first determining step and the second determining step it is determined that coughing has occurred, it is determined that coughing has been detected.
